# FASCICULE DE BREVET EUROPEEN

(11) **EP 4 504 348 B1**
(45) Date de publication et mention de la délivrance du brevet: **08.07.2026**
(21) Numéro de dépôt: 23769203.3
(22) Date de dépôt: 13.09.2023
(51) Int. Cl.: A61Q 19/06, A61K 8/99, A61K 8/34

(54) **UTILISATION COSMÉTIQUE D'UN EXTRAIT DE PSEUDOALTEROMONAS PARAGORGICOLA COMME AGENT AMINCISSANT**
KOSMETISCHE VERWENDUNG EINES EXTRAKTS VON PSEUDOALTEROMONAS PARAGORGICOLA ALS SCHLANKHEITSMITTEL.
COSMETIC USE OF AN EXTRACT OF PSEUDOALTEROMONAS PARAGORGICOLA AS A SLIMMING AGENT

(30) Priorité: 14.09.2022 FR 2209247
(43) Date de publication de la demande: 12.02.2025
(73) Titulaire: Laboratoires Clarins, 75017 Paris (FR)
(72) Inventeur: FAVRE-MERCURET, Magali, 95300 PONTOISE (FR); FITOUSSI, Richard, 78600 MAISONS-LAFFITTE (FR); BEAUCHEF, Gallic, 78110 Le Vesinet (FR)
(74) Mandataire: Gevers & Orès
(86) Numéro de dépôt international: PCT/EP2023/075105
(87) Numéro de publication internationale: WO 2024/056709

(56) Documents cités:
- WO-A1-2005/087189
- WO-A1-2018/207988
- FR-A1- 2 921 267
- FR-B1- 2 849 595

## Description

La présente invention se rapporte au domaine du traitement cosmétique de la peau. Elle concerne une composition cosmétique comprenant un extrait de *Pseudoalteromonas paragorgicola.*

La peau est un organe de revêtement superficiel qui représente une barrière de protection relativement efficace vis-à-vis des agressions externes et recouvrant l'intégralité de la surface corporelle. Elle est souvent présentée comme l'organe le plus lourd du corps humain. De plus, la peau joue également un rôle actif dans de nombreux processus biologiques et biochimiques.

Elle est constituée de trois couches ou tissus superposés comprenant de la partie la plus superficielle à la plus profonde, l'épiderme, le derme et l'hypoderme.

Ce dernier également appelé tissu adipeux représente 15 à 20% du poids corporel chez un individu de poids moyen. Il est rattaché à la partie inférieure du derme par des expansions de fibres de collagène. L'épaisseur de l'hypoderme est amenée à varier suivant l'âge, la localisation corporelle et le sexe, et assure en grande partie le modelage des courbes du corps.

En plus d'être un tissu graisseux vascularisé contenant des adipocytes dont le rôle est d'absorber les graisses pour les stocker sous forme de triglycérides, c'est un organe de réserve dans lequel les graisses peuvent être mobilisées et transformées en énergie en cas d'effort prolongé de l'organisme. Il sert également d'organe de protection, mécanique contre les chocs et thermique vis-à-vis des variations de températures afin de maintenir l'homéostasie corporelle. Il contrôle la mobilisation des lipides par deux activités métaboliques principales : la lipogenèse (synthèse des acides gras (AG) et triglycérides, et stockage du surplus d'énergie) et la lipolyse (hydrolyse des triglycérides en cas de besoin énergétique).

Le tissu adipeux n'est pas uniquement un organe de stockage dont l'excès de développement est cause d'obésité et des troubles métaboliques qui l'accompagnent comme le diabète de type Il ; il est également un véritable organe endocrine. Cette fonction lui a été attribuée il y a maintenant plus de 20 ans par la découverte de la leptine, une hormone secrétée par le tissu adipeux en réponse à la prise alimentaire et inhibant l'appétit en régulant les circuits neuronaux localisés dans le cerveau.

Le tissu adipeux est un organe majeur dans le maintien de l'homéostasie énergétique de l'organisme par ses capacités de stockage et de libération des acides gras mais également par ses fonctions de sécrétion. Les cellules endothéliales des réseaux vasculaires du tissu adipeux constituent une barrière physique appelé endothélium entre le compartiment sanguin et les adipocytes. Les cellules endothéliales des réseaux vasculaires du tissu adipeux jouent un rôle majeur dans le contrôle des échanges de nutriments, d'hormones, d'oxygène et de cellules immunitaires et inflammatoires. Ces échanges et leurs régulations sont déterminants et spécifiques du statut métabolique du tissu adipeux.

La place de l'endothélium dans la biologie des dépôts adipeux a considérablement évolué depuis sa vision historique d'une couche inerte de cellules servant de revêtement interne au système circulatoire peu développé dans la masse grasse. Les données actuelles montrent d'une part que le réseau vasculaire est très étendu dans les dépôts adipeux et d'autre part qu'il joue un rôle clé dans le développement et la fonction des tissus adipeux. De plus, l'hétérogénéité remarquable de la couche endothéliale est maintenant bien reconnue, qu'il s'agisse des différences en fonction de l'emplacement des vaisseaux conducteurs (artères, capillaires et veines) ou de l'hétérogénéité fonctionnelle dans le contexte d'organes spécifiques, tels que le cœur, le foie et le tissu adipeux.

Une telle diversité de l'endothélium est la clé des rôles spécialisés de sa fonction, y compris la perméabilité, le trafic des leucocytes et l'hémostase mais également le transport de l'oxygène, des fluides et des nutriments de la circulation vers les tissus cibles. Le concept de transport des acides gras (AG) par l'endothélium existe depuis de nombreuses années, mais il est passé de transfert passif et diffus à un processus actif, hautement régulé et ce de manière tissu-spécifique et qui implique des voies de signalisation multiples et complexes. (Hagberg, C.E., et al., Vascular endothelial growth factor B controls endothelial fatty acid uptake. Nature, 2010. 464(7290): p.917-21). Les AG, naturellement stockés sous forme de triglycérides dans les adipocytes, doivent être canalisés à travers l'endothélium microvasculaire selon des mécanismes encore mal connus. Le transport des AG à travers l'endothélium microvasculaire non fenestré implique des transporteurs membranaires FATP (fatty-acid-transport proteins ou protéines de transport des acides gras) et des transporteurs cytoplasmiques FABP (fatty-acid-binding proteins ou protéines de liaison aux acides gras). Dans l'endothélium du tissu adipeux blanc (WAT ou white adipose tissue), d'autres facteurs, dont le PPARG (récepteur gamma activé par les proliférateurs de peroxysomes), ont été proposés pour jouer un rôle.

Pour analyser le transport des AG dans les cellules endothéliales du tissu adipeux humain (hATEC ou human adipose tissue endothelial cells), l'équipe de Anaïs Briot a mesuré la capacité de cellules endothéliales primaires fraîchement isolées de tissu adipeux à absorber un AG fluorescent (Bodipy FL C16) in vitro. A 37°C, les cellules incorporent efficacement les AG fluorescents. En présence de rosiglitazone agoniste du PPARG à 37°C, une augmentation de l'absorption des AG fluorescents a été observée démontrant que le traitement à la rosiglitazone favorise l'absorption des AG dans les cellules endothéliales du tissu adipeux humain (Briot, A., et al., Senescence Alters PPARgamma (Peroxisome Proliferator-Activated Receptor Gamma)-Dependent Fatty Acid Handling in Human Adipose Tissue Microvascular Endothelial Cells and Favors Inflammation. Arterioscler Thromb Vasc Biol, 2018. 38(5): p.1134-1146).

Ces mécanismes de transports actifs des acides gras par les cellules endothéliales peuvent donc être une cible pour freiner l'entrée des acides gras dans les dépôts graisseux et vers les adipocytes et ainsi pour limiter leurs stockages dans les masses grasses.

Ceci a permis la mise au point d'un modèle cellulaire de cellules endothéliales microvasculaires de tissus adipeux sous-cutané abdominal humain immortalisées grâce auquel la Demanderesse a mis en évidence l'effet anti-hyperplasie d'un extrait de *Pseudoalteromonas paragorgicola.* En effet, l'extrait de *Pseudoalteromonas paragorgicola* limite le transport d'acides gras fluorescents à chaine longue C16 (Bodipy FL C16), ce qui lui permet de limiter le stockage des AG dans les adipocytes et d'exercer une action préventive sur l'augmentation du tissu adipeux sous-cutané.

De par ses propriétés, l'extrait de *Pseudoalteromonas paragorgicola* apparait donc être un actif utile comme agent amincissant.

Divers documents de l'état de la technique proposent des agents amincissants cosmétiques : le document FR2849595 décrit l'utilisation de la plante *Bocoa prouacensis* comme agent amincissant ; le document FR2921267 propose d'utiliser un activateur de la périlipine tel qu'un extrait de plante de la famille des Dipsacaceaes dans une composition cosmétique amincissante ; ou encore le document WO2005/087189 décrit l'utilisation d'un inhibiteur de métalloprotéinase 2 et/ou 9 tel qu'un extrait de *Baccharis genistelloides* dans une composition cosmétique amincissante. Aucun ne suggère l'effet amincissant d'un extrait de *Pseudoalteromonas paragorgicola.*

L'invention concerne donc l'utilisation cosmétique d'un extrait de *Pseudoalteromonas paragorgicola* comme agent amincissant.

On entend par utilisation cosmétique l'utilisation d'une composition cosmétique, c'est-à-dire une formulation convenant à une application cutanée sur peau saine, comprenant l'extrait de *Pseudoalteromonas paragorgicola* ainsi que des excipients cosmétiquement acceptables.

L'extrait de *Pseudoalteromonas paragorgicola* peut également être avantageusement utilisé dans une composition amincissante.

L'utilisation selon l'invention est particulièrement adaptée à une application topique sur des peaux saines. Dans le cadre de la présente invention, on désigne par peau saine une peau qui ne présente pas de pathologie cutanée et qui en outre est en bon état (au sens de l'article 2.1.a) du RÈGLEMENT (CE) No1223/2009 DU PARLEMENT EUROPÉEN ET DU CONSEIL du 30 novembre 2009 relatif aux produits cosmétiques). On entend donc par utilisation cosmétique une utilisation non thérapeutique.

L'utilisation selon l'invention est particulièrement adaptée à une application topique sur la peau du visage aussi bien qu'à une application sur la peau du corps.

*Pseudoalteromonas paragorgicola* est une bactérie qui fait partie de l'ordre des Alteromonadales, et plus précisément de la famille des Pseudolateromonadaceae.

C'est une bactérie de couleur orange-pâle, de type Gram négatif en forme de « bâtonnet ». Elle se développe en aérobie stricte. Elle mesure 0,7 à 0,9 µm de diamètre et 1 à 1,2 µm de long avec un flagelle polaire unique. Son ADN comporte 41% de guanine et cytosine. La colonie, ronde, mesure 2 à 3 mm de diamètre et est légèrement orangée.

Cette bactérie a la capacité de dégrader la gélatine, l'ADN et le polysorbate 80. Elle peut se développer à des concentrations en sel (NaCl) de 1 à 6% ou dans de l'eau de mer. La gamme de température lui permettant de se multiplier est comprise entre 2 et 30°C avec une température optimale de 25°C. Cette bactérie peut se développer à des pH entre 6 et 10, son optimum étant à pH 7,5-8.

Elle a été isolée pour la première fois en 1988 sur le prélèvement d'un corail prélevé à 202 m de profondeur, dans une eau à 10°C et avec une salinité de 32 ‰, à la suite d'une expédition réalisée dans l'Océan Pacifique, et plus précisément au large de l'ile Onekotan (archipel des îles Kouriles, Russie). Elle a également été récoltée lors d'une expédition réalisée dans les eaux internationales proche du Groenland (MEDEA II, juin à juillet 2012). Le prélèvement ayant permis de l'isoler a été effectué à 2 700 m de profondeur, dans une eau à 2,98°C.

Certains antibiotiques (ampicilline, carbénicilline, polymixine, streptomycine) sont efficaces sur cette espèce.

Le nom donné à l'espèce est formé de « Paragorgia », un genre de la famille des Gorgonaceae et du suffixe latin « -cola » signifiant « qui habite ». En effet, l'espèce a été isolée sur le corail *Paragorgia arborea.*

Aucun usage traditionnel n'est recensé à ce jour pour cette bactérie.

Selon un mode particulier de l'invention l'extrait est obtenu selon un procédé comprenant au moins les étapes suivantes, l'homme du métier sait mettre en œuvre un procédé d'extraction à partir du micro-organisme :
- Fermentation
- Extraction de préférence aqueuse à chaud
- Centrifugation
- Filtrations successives
- Dialyse
- Filtration
- Stabilisation par ajout de 20% Propanediol + 0,6% gomme xanthane + 0,5% Glyceryl Caprylate.

L'extrait de *Pseudoalteromonas paragorgicola* ainsi obtenu est un liquide gris à jaune (de type gel opaque), d'odeur caractéristique. Il présente les caractéristiques analytiques suivantes :
- pH = 4 - 6
- densité à 20°C = 1.00 - 1.04 (g/ml)
- matière sèche = 0.25 - 0.75 %
- indice de réfraction = 1.34 - 1.38
- poids moléculaire = 5-250 kDa

L'utilisation cosmétique selon la présente invention met préférentiellement en œuvre un extrait de *Pseudoalteromonas paragorgicola* ou une composition cosmétique comprenant un tel extrait.

L'extrait selon l'invention est un extrait du micro-organisme *Pseudoalteromonas paragorgicola,* plus particulièrement ledit extrait est un extrait hydrosoluble.

De façon avantageuse, l'extrait du micro-organisme *Pseudoalteromonas paragorgicola* est un extrait hydrosoluble obtenu par fermentation puis extraction aqueuse à chaud, filtrations purifiantes et stabilisé par du propanediol.

De préférence, la composition cosmétique selon l'invention comprend de 0,01 à 10% d'un extrait de *Pseudoalteromonas paragorgicola* par rapport au poids total de la composition. Avantageusement, la composition comprend de 0,01 à 5% d'un extrait de *Pseudoalteromonas paragorgicola* en poids par rapport au poids total de la composition. Les compositions selon l'invention peuvent comprendre un ou plusieurs agents de formulation ou additifs d'usage connu et classique dans les compositions cosmétiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres UV, etc. Grâce à ses connaissances en matière de cosmétique, l'homme du métier saura quels agents de formulation ajouter aux compositions de l'invention et en quelles quantités en fonction des propriétés recherchées.

Les compositions selon l'invention peuvent se présenter sous toute forme connue de l'homme du métier dans le domaine de la cosmétologie sans autre restriction galénique que l'application sur le visage et sur le corps. De façon avantageuse, les compositions selon l'invention se présentent sous la forme d'un gel, d'une crème, d'une lotion, d'un masque, d'une huile, d'un lait, d'un spray, etc.

L'invention concerne également l'utilisation d'un extrait de *Pseudoalteromonas paragorgicola* pour tonifier et/ou raffermir la peau, diminuer le capiton, affiner et/ou redéfinir la silhouette, réduire les amas graisseux sous cutanés, prévenir et/ou retarder les manifestations cutanées de l'augmentation du tissu adipeux que sont la cellulite et/ou les capitons.

Les exemples ci-après concernent, d'une part l'impact de l'actif *Pseudoalteromonas paragorgicola* sur le transport d'acides gras (à chaine longue C16) sur modèle cellulaire (cellules endothéliales microvasculaires de tissus adipeux sous-cutané abdominal humain immortalisées). D'autre part, des exemples de formulations utilisant l'extrait *Pseudoalteromonas paragorgicola* objet de la présente invention.

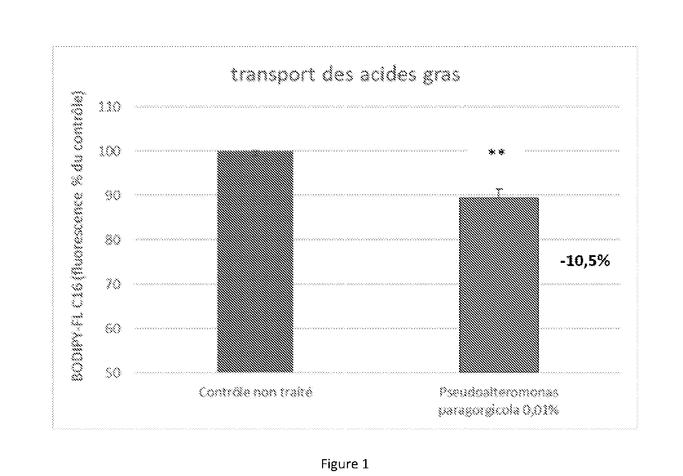

Figure 1 : Les exemples font référence à la figure 1 qui montre l'effet d'un extrait de Pseudoalteromonas paragorgicola tel que décrit précédemment sur le transport d'acides gras C16 fluorescent. Cette Figure représente la moyenne +/- l'écart standard à la moyenne des 8 mesures en duplicate des cellules traitées avec l'extrait de *Pseudoalteromonas paragorgicola à* la dose 0,01% rapportée en pourcentage des cellules non traitées (contrôle).

### EXEMPLES

### I. Mise en évidence du rôle de Pseudoalteromonas paragorgicola sur le transport des acides gras (AG)

### A. Matériels et Méthodes

### 1. Culture cellulaire

Les cellules endothéliales microvasculaires (CD45-/CD34+/CD31+) de tissus adipeux humain immortalisées sont ensemencées à confluence (150000 cellules /cm2) sur des plaques avec un revêtement (coating) à la fibronectine (2 µg/cm2, 30 minutes, température ambiante) permettant leur adhésion. Les cellules sont maintenues en milieu de croissance (ECGM MV ou endothelial cell growth medium MV ; PromoCell C-22020) à 37°C sous une atmosphère de 5% CO₂. Les cellules sont ensuite rincées au PBS puis traitées ou non en duplicat avec l'extrait de *Pseudoalteromonas paragorgicola à* 0,01% (concentration étudiée non cytotoxique) dans le milieu de croissance ECGM MV pendant 24h.

### 2. Transport des acides gras fluorescents

Les cellules non traitées (contrôle) ou traitées avec l'extrait de *Pseudoalteromonas paragorgicola à* la dose 0,01% sont incubées dans un milieu de culture minimum constitué d'une solution saline équilibrée de Hank (HBSS ou Hank's Balanced Salt Solution) avec 0,1% d'albumine de sérum bovin (BSA ou bovine serum albumin) sans acides gras pendant 1 heure. Le milieu est ensuite éliminé et remplacé par le milieu minimum supplémenté avec un AG fluorescent (BODIPY-FL C16) (Thermofisher Réf D3821 ; 750 nM). Les cellules sont incubées pendant 1 heure. Après lavage avec un tampon phosphate salin ou PBS (phosphate buffer saline) préchauffé à 37°C, les cellules sont fixées avec une solution de PBS contenant 4% de paraformaldéhyde ou PFA pendant 10 minutes à température ambiante et à l'abri de la lumière. L'intensité de fluorescence est mesurée à l'aide d'un fluoromètre TECAN Infinite F500, avec une lecture à la longueur d'onde 520 nm. 8 champs par puits sont mesurés en duplicate. L'analyse statistique est réalisée avec un test de Student apparié.

### 3. Toxicité

Pour évaluer la toxicité cellulaire, les cellules sont fixées dans un tampon phosphate salin ou PBS contenant 4% de paraformaldéhyde, pendant 10 minutes à température ambiante à l'abri de la lumière puis rincées au PBS. Les cellules sont marquées au DAPI ou 4',6-diamidino-2-phénylindole (20 µg/mL (Invitrogen D1306)) pendant 30 minutes puis rincées au PBS à 4°C à l'abri de la lumière. La coloration au DAPI permet de dénombrer les cellules viables en détectant par fluorescence les noyaux ensuite quantifiés par imagerie. L'intensité de fluorescence est mesurée à l'aide d'un fluoromètre TECAN Infinite F500, avec une lecture à 448 nm. 8 champs par puits sont mesurés en duplicate.

### B. Résultats et conclusion

Dans les conditions expérimentales retenues, l'extrait de *Pseudoalteromonas paragorgicola à* 0,01% n'a pas induit de toxicité sur les cellules endothéliales microvasculaires des dépôts adipeux humains traitées pendant 24 heures.

Dans les conditions expérimentales retenues, le pré-traitement des cellules endothéliales microvasculaires des dépôts adipeux humains par l'extrait de *Pseudoalteromonas paragorgicola à* 0,01% inhibe le transport de l'acide gras C16. En effet, l'absorption de l'acide gras C16 fluorescent (BODIPY-FL C16) par les cellules diminue significativement dans la condition traitée par l'extrait de *Pseudoalteromonas paragorgicola* comparativement à la condition non traitée (contrôle) (figure 1). Le pré-traitement des cellules endothéliales microvasculaires des dépôts adipeux humains par l'extrait de *Pseudoalteromonas paragorgicola à* 0,01% inhibe significativement (**, p-value = 0,0047) de 10,5% le transport des acides gras C16.

### II. Exemple de formulation

| EMULSION | (%) |
|---|---|
| ISONONYL ISONONANOATE | 3,50 |
| DICAPRYLYL CARBONATE / TOCOPHEROL | 1,50 |
| C14-22 ALCOHOLS / C12-20 ALKYL GLUCOSIDE | 3,00 |
| CETYL ETHYLHEXANOATE | 3,50 |
| SODIUM ACRYLATES COPOLYMER / PHOSPHOLIPIDS / HYDROGENATED POLYDECENE / POLYGLYCERYL-10 STEARATE / HELIANTHUS ANNUUS (SUNFLOWER) SEED OIL / TOCOPHEROL | 2,50 |
| AQUA | 65,30 |
| CAFFEINE | 1,80 |
| BUTYLENE GLYCOL | 1,50 |
| SODIUM POLYACRYLATE | 0,20 |
| GLYCERIN | 4,00 |
| POTASSIUM CETYL PHOSPHATE | 0,50 |
| ESCIN | 0,20 |
| AQUA / METHYLSILANOL MANNURONATE / SORBIC ACID / PHENOXYETHANOL | 2,00 |
| AQUA / PROPANEDIOL / XANTHAN GUM / GLYCERYL CAPRYLATE / PSEUDOALTEROMONAS FERMENT EXTRACT | 0,10 |
| PARFUM | 0,40 |
| ALCOHOL | 10,00 |

| GEL | (%) |
|---|---|
| AQUA | 88,37 |
| CAFFEINE | 0,50 |
| GLYCERIN | 4,00 |
| ETHYLHEXYLGLYCERIN | 0,20 |
| PROPANEDIOL | 1,50 |
| PENTYLENE GLYCOL | 1,00 |
| AMMONIUM ACRYLOYLDIMETHYLTAURATE/VP COPOLYMER | 0,70 |
| POLYACRYLATE CROSSPOLYMER-6 / AQUA / t-BUTYL ALCOHOL | 0,35 |
| ESCIN | 0,10 |
| AQUA | 0,08 |
| SODIUM HYDROXIDE | 0,10 |
| AQUA / AVENA SATIVA KERNEL EXTRACT / SODIUM BENZOATE | 2,00 |
| AQUA / PROPANEDIOL / XANTHAN GUM / GLYCERYL CAPRYLATE / PSEUDOALTEROMONAS FERMENT EXTRACT | 0,10 |
| GLYCERIN / AQUA / PANAX GINSENG ROOT EXTRACT | 1,00. |

## Revendications

1. Utilisation cosmétique d'un extrait de *Pseudoalteromonas paragorgicola* comme agent amincissant.

2. Utilisation cosmétique d'un extrait de *Pseudoalteromonas paragorgicola* dans une composition amincissante.

3. Utilisation selon l'une quelconque des revendications précédentes, dans laquelle l'extrait de *Pseudoalteromonas paragorgicola* est un extrait hydrosoluble obtenu par fermentation.

4. Utilisation selon l'une quelconque des revendications précédentes, dans laquelle l'extrait de *Pseudoalteromonas paragorgicola* est stabilisé par du propanediol.

5. Utilisation selon l'une quelconque des revendications précédentes, **caractérisé en ce que** ledit extrait est formulé dans une composition cosmétique qui comprend de 0,01 à 10% d'extrait de *Pseudoalteromonas paragorgicola* en poids par rapport au poids total de la composition, de préférence 0,01 à 5% dudit extrait en poids par rapport au poids total de la composition.

6. Utilisation selon la revendication 2 ou 5, dans laquelle ladite composition comprend en outre un ou plusieurs agents de formulation ou additifs d'usage connu et classique dans les compositions cosmétiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres.

7. Utilisation selon l'une quelconque des revendications précédentes pour tonifier et/ou raffermir la peau, diminuer le capiton, affiner et/ou redéfinir la silhouette, réduire les amas graisseux sous cutanés, prévenir et/ou retarder les manifestations cutanées de l'augmentation du tissu adipeux que sont la cellulite et/ou les capitons.

## Patentansprüche

1. Kosmetische Verwendung eines Extrakts von *Pseudoalteromonas paragorgicola* als Schlankheitsmittel.

2. Kosmetische Verwendung eines Extrakts von *Pseudoalteromonas paragorgicola* in einer schlankmachenden Zusammensetzung.

3. Verwendung nach einem der vorstehenden Ansprüche, wobei der Extrakt von *Pseudoalteromonas paragorgicola* ein durch Fermentation erhaltener wasserlöslicher Extrakt ist.

4. Verwendung nach einem der vorstehenden Ansprüche, wobei der Extrakt von *Pseudoalteromonas paragorgicola* durch Propandiol stabilisiert wird.

5. Verwendung nach einem der vorstehenden Ansprüche, **dadurch gekennzeichnet, dass** der Extrakt in eine kosmetische Zusammensetzung formuliert wird, die bezogen auf das Gesamtgewicht der Zusammensetzung zu 0,01 bis 10 Gew.-% Extrakt von *Pseudoalteromonas paragorgicola* enthält, vorzugsweise 0,01 bis 5 Gew.-% bezogen auf das Gesamtgewicht der Zusammensetzung.

6. Verwendung nach Anspruch 2 oder 5, wobei die Zusammensetzung weiter ein oder mehrere übliche bekannte und in kosmetischen Zusammensetzungen klassische Formulierungsmittel oder Zusatzstoffe umfasst, wie, beispielhaft und auf nicht einschränkende Weise, Weichmacher, Farbstoffe, filmbildende Wirkstoffe, Tenside, Parfums, Konservierungsmittel, Emulgatoren, Öle, Glycole, Vitamine wie Vitamin E, Filter.

7. Verwendung nach einem der vorstehenden Ansprüche zum Straffen und/oder Festigen der Haut, Verringern von Fettpölsterchen, Verfeinern oder neu Definieren der Silhouette, Reduzieren subkutaner Fettablagerungen, Vorbeugen oder Verzögern von kutanen Erscheinungen von Fettgewebeerhöhungen, die Cellulite oder Fettpölsterchen sind.

## Claims

1. A cosmetic use of an extract of *Pseudoalteromonas paragorgicola* as a slimming agent.

2. The cosmetic use of an extract of *Pseudoalteromonas paragorgicola* in a slimming composition.

3. The use according to any of the preceding claims, wherein the *Pseudoalteromonas paragorgicola* extract is a water-soluble extract obtained by fermentation.

4. The use according to any of the preceding claims, wherein the *Pseudoalteromonas paragorgicola* extract is stabilized with propanediol.

5. The use according to any of the preceding claims, **characterized in that** said extract is formulated in a cosmetic composition comprising from 0.01 to 10% *Pseudoalteromonas paragorgicola* extract by weight relative to the total weight of the composition, preferably from 0.01 to 5% of said extract by weight relative to the total weight of the composition.

6. The use according to claim 2 or 5, wherein said composition further comprises one or more formulation agents or additives of known and conventional use in cosmetic compositions such as, by way of example and without limitation, emollients, colorants, film-forming agents, surfactants, fragrances, preservatives, emulsifiers, oils, glycols, vitamins such as vitamin E, and filters.

7. The use according to any of the preceding claims to tone and/or firm the skin, reduce dimpling, refine and/or redefine the silhouette, reduce subcutaneous fat deposits, and prevent and/or delay the skin manifestations of increased adipose tissue, namely cellulite and/or dimpling.
